# FASCICULE DE BREVET EUROPEEN

(11) **EP 1 229 895 B1**
(45) Date de publication et mention de la délivrance du brevet: **21.12.2011**
(21) Numéro de dépôt: 00974628.0
(22) Date de dépôt: 02.11.2000
(51) Int. Cl.: A61K 8/99, A61Q 19/06

(54) **COMPOSITION AMINCISSANTE CONTENANT UNE SUBSTANCE INDUCTEUR DE LA PRODUCTION D'IL-6**
KOSMETISCHES SCHLANKHEITSMITTEL ENTHALTEND EINE SUBSTANZ DIE DIE PRODUKTION VON IL-6 INDUZIERT
SLIMMING COMPOSITION CONTAINING A SUBSTANCE INDUCING IL-6 PRODUCTION

(30) Priorité: 05.11.1999 FR 9913917
(43) Date de publication de la demande: 14.08.2002
(73) Titulaire: SANOFI, 75013 Paris (FR)
(72) Inventeur: CASELLAS, Pierre, F-34090 Montpellier (FR); DEROCQ, Jean, Marie, F-34570 Murviel les Montpellier (FR); GUESNET, Joelle, F-91440 Bures sur Yvette (FR)
(74) Mandataire: Monain, Patrice
(86) Numéro de dépôt international: PCT/FR2000/003048
(87) Numéro de publication internationale: WO 2001/032137

(56) Documents cités:
- EP-A- 0 493 151
- EP-A- 0 838 217
- WO-A-99/40896
- FR-A- 2 714 598
- FR-A- 2 758 724

## Description

La présente invention concerne des compositions cosmétiques contenant des substances actives amincissantes.

Les substances actives présentes dans la composition cosmétique sont choisies parmi un antagoniste des récepteurs du neuropeptide Y ci-après dénommé NPY, un antagoniste des récepteurs α2 et un inducteur de la production d'interleukine-6 ci-après dénommée IL-6.

La présente invention se rapporte ainsi à une composition cosmétique amincissante contenant au moins un composant inducteur de la production d'IL-6 par les adipocytes, un composant α₂-antagoniste, un composant NPY antagoniste en mélange avec un excipient pour préparations cosmétiques.

Le NPY antagoniste, l'α2 antagoniste ou l'inducteur de la production d'IL-6 est un produit obtenu par fermentation d'un micro-organisme, par exemple d'une bactérie ou d'un champignon.

Le composant inducteur de la production d'IL-6 par les adipocytes est susceptible d'être obtenu par fermentation d'une souche de *Rhodotorula sp,* notamment celle déposée auprès de la C.N.C.M. de l'institut Pasteur sous le numéro 1-1844, ou de ses mutants producteurs.

Le composant α₂-antagoniste est susceptible d'être obtenu par fermentation de la souche *Bacillus licheniformis,* déposée auprès de la C.N.C.M. de l'Institut Pasteur sous le numéro I 1778, ou un de ses mutants producteurs.

Le composant NPY-antagoniste est susceptible d'être obtenu par fermentation de la souche *Streptomyces sp.,* déposée auprès de la C.N.C.M. de l'Institut Pasteur sous le numéro I 1332, ou un de ses mutants producteurs.

La demande de brevet EP 838217 décrit une composition cosmétique amincissante qui contient un NPY antagoniste et un α2 antagoniste. Les substances actives de cette composition sont obtenues par fermentation de deux micro-organismes déposés auprès de le C.N.C.M. de l'Institut Pasteur où ils ont été enregistrés respectivement sous les références I 1332 et I 1778. Dans la présente description, ces substances actives seront nommées respectivement substance A et substance B. La composition cosmétique amincissante les contenant sera nommée composition Am1.

Cette composition amincissante Am1 agit au niveau du tissu adipeux sous-cutané et contrôle la libération des graisses stockées dans les adipocytes.

En bloquant les récepteurs α2 et NPY, la composition Am1 permet de désengorger les adipocytes hypertrophiés et d'éviter tout nouveau surstockage des adipocytes en permettant l'expression des récepteurs β dont l'activité prolipolytique est normalement masquée par les récepteurs antilipolytiques α2 et NPY largement excédentaires dans les tissus adipeux sous-cutanés. La composition Am1, permet ainsi d'agir efficacement sur la sortie des acides gras stockés dans les adipocytes.

Les compositions selon la présente invention maintiennent cette activité mais la complètent d'une part par une action de ralentissement sur l'entrée des acides gras qui s'exerce par un effet réducteur sur la production de la LPL, l'enzyme responsable de l'entrée des acides gras dans la cellule et d'autre part sur l'hyperplasie des adipocytes. Cet effet inattendu complémentaire est obtenu par une substance capable d'induire la production d'IL-6 par les adipocytes. Cette substance est produite par un micro-organisme déposé auprès de la C.N.C.M. de l'Institut Pasteur enregistré sous la référence I 1844. Cette substance active sera nommée ci-après substance C. Longtemps considérés uniquement pour leur rôle de réserve énergétique, les adipocytes ont mis en évidence au cours de ces dernières années des fonctions de cellule endocrine et sécrétrice (Ailhaud G. et al. Médecine/Science 1998, 14, 858-864). La leptine illustre parfaitement cette nouvelle fonction sécrétoire de l'adipocyte. Cette protéine est produite par l'adipocyte mature de façon spécifique. Elle est impliquée dans le contrôle de la satiété, elle serait également impliquée dans la régulation des dépôts graisseux. L'adipocyte assure ainsi la production de facteurs à activité autocrine et paracrine qui vont moduler la physiologie du tissu adipeux.

En particulier, il a été démontré très récemment que la fonction endocrine de l'adipocyte s'exerçait sur deux médiateurs spécifiques :
- l'acide lysophosphatidique (LPA), un facteur lipidique dont la production est médiée par l'activation du récepteur α2 et dont la fonction consiste à recruter de nouveaux adipocytes (Valet P. et al. J. Clin. Invest. 1998, 101 (7), 1431-1438).
- l'lL-6, qui est une cytokine multifonctionnelle.

La substance C est connue en tant qu'inducteur de la production d'IL-6 par les kératinocytes. Son procédé d'obtention est décrit dans la demande de brevet internationale WO 99/40896. Elle exerce un effet sur le vieillissement cutané.

Rien ne pouvait indiquer que cette substance (produite par le micro-organisme I 1844) aurait par ailleurs, exercé une double action sur les adipocytes.

Récemment des études ont montré que les cellules adipeuses pouvaient produire l'IL-6 dont la fonction au niveau adipocytaire consiste à réprimer la synthèse de la LPL, l'enzyme responsable de l'entrée des acides gras dans la cellule adipeuse (Greenberg A.S. et al. Cancer Research 1992, 52, 4113-4116).

La production d'IL-6 par les cellules du tissu adipeux sous-cutané est positivement corrélée avec une augmentation des index d'obésité (Mohamed Ali V. et al. J. Clin. Endocrinol. Metab. 1997, 82, 4196-4200) mais diffère également en fonction de l'origine du tissu adipeux.

Ainsi, chez les sujets obèses, les adipocytes profonds prélevés dans la région omentale libèrent 2 à 3 fois plus d'IL-6 que les adipocytes sous-cutanés (Fried S.K. et al. J. Clin. Endocrinol. Metab. 1998, 83, 847-860).

Enfin, en plus de la capacité de l'IL-6 à réduire la production de la LPL par les adipocytes, l'IL-6 pourrait aussi agir par inhibition sur la différenciation des cellules précurseurs des adipocytes (Hauner H. et al. 8th International Congress on Obesity 1999, 47-53).

L'IL-6 apparaît donc comme une cytokine qui agit de façon autocrine (sur la LPL) et paracrine (sur les pré-adipocytes) pour freiner le développement du tissu adipeux sous-cutané.

Cependant, comme toutes les cytokines, l'IL-6 est une glycoprotéine pléïotropique c'est-à-dire qu'elle présente des effets différents en fonction de la cellule qui la produit. Ainsi, au niveau de l'adipocyte, l'IL-6 aura des effets de toute autre nature que ceux déjà connus avec le kératinocyte.

En particulier, dans l'adipocyte, l'IL-6 synthétisée par la cellule régule à la baisse la teneur et donc l'activité de l'enzyme qui assure l'entrée des graisses.

Ainsi, les compositions selon l'invention permettent de contrôler les phénomènes d'entrée et de sortie des acides gras dont le stockage et l'hypertrophie des adipocytes. Elle permet également d'agir sur l'hyperplasie des adipocytes.

II est connu que la formation des nouveaux adipocytes intervient chez l'homme, tout au long de la vie des individus. Ainsi des cellules graisseuses "dormantes" ont été isolés chez des sujets âgés quel que soit leur sexe (Ailhaud G. et al. Int. J. Obes. Relat. Metab. Disord. 1992, 16(2), 517-521 ; Spiegelman B. et al. Cell 1996, 87, 377-389 ; Hauner et al. J. Clin. Invest. 1989; 84, 1663-1670).

Le développement du tissu adipeux résulte à la fois d'une augmentation de la taille des adipocytes consécutive à l'accumulation excessive des acides gras, et du recrutement de nouvelles cellules graisseuses provenant de la multiplication et de la différenciation de cellules précurseurs d'adipocytes, les pré-adipocytes (Valet P. et al. J. Clin. Invest. 1998, 101(7), 1431-1438).

Ce recrutement de nouveaux adipocytes est médié par des facteurs solubles produits par les adipocytes hypertrophiés ; en particulier, par le LPA *via* la stimulation du récepteur α2, et par l'IL-6 *via* son pouvoir de contrôle sur la différenciation pré-adipocytaire.

Le blocage du récepteur α2 est donc de nature à permettre, en parallèle de son bénéfice sur la lipolyse, de réprimer la libération du LPA, avec pour avantage une inhibition du recrutement des nouveaux adipocytes.

L'IL-6 limite la maturation des pré-adipocytes en adipocytes différenciés. La stimulation adipocytaire de la production de cette cytokine par l'inducteur d'IL-6 renforce cette propriété.

L'association de la substance B inhibiteur du récepteur et de la substance C, inducteur d'IL-6, est donc de nature à permettre une efficacité accrue et originale sur l'hyperplasie des cellules graisseuses. L'addition de la substance A à cette association permet d'obtenir une composition amincissante intervenant sur plusieurs mécanismes d'action à savoir l'hypertrophie et l'hyperplasie.

Des études ont été réalisées pour mettre en évidence l'activité de la substance active obtenue à partir du micro-organisme I 1844, ou substance C, sur les adipocytes matures et sur la différenciation adipocytaire.

On a maintenant, selon l'invention, préparé une composition cosmétique amincissante qui contient les substances A, B et C et qui sera nommée ci-après Am2.

L'effet amincissant de Am2 a fait l'objet d'études cliniques.

Les effets biologiques *in vitro* de la substance C ont été étudiés sur la lignée murine 3T3-L1. Cette lignée pré-adipocytaire, cultivée en routine dans du milieu DMEM (Dubelco's Minimum Essential Medium) enrichi de 10% de sérum de veau foetal présente la propriété de se différencier en adipocytes matures en présence d'inducteurs tels que l'insuline et l'indométhacine (Slieker L. J. Biochem. Biophys. Res. Com. 1998, 251, 225-229). Huit à dix jours après l'induction de la différenciation , les cellules 3T3-L1 présentent les caractéristiques d'adipocytes matures. Ce passage du stade de cellule immature au stade d'adipocyte fonctionnel peut être apprécié morphologiquement par l'apparition de vésicules de stockage lipidique intra-cytoplasmiques, visibles au microscope, ou détectables biochimiquement par la mesure de la production de leptine, hormone de satiété uniquement produite par des adipocytes matures.

L'influence de la substance C sur l'activité adipocytaire a été étudiée à deux niveaux. D'une part en mesurant la capacité de cet actif à moduler la production d'IL-6 adipocytaire et d'autre part en évaluant son effet sur la différenciation en adipocytes matures.

### 1- Effet de la substance C sur la production d'IL-6 par les adipocytes matures 3T3-L1.

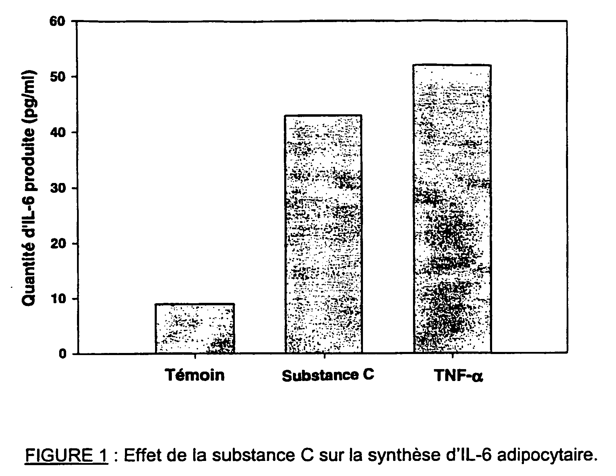

Des adipocytes matures 3T3-L1 (récoltés après 10 jours de différenciation induite par l'association insuline (5 µg/ml) et indométhacine (125 µM) sont stimulés par la substance C à 1% (v/v) pendant 24 heures. Le surnageant de culture est ensuite prélevé et son contenu en IL-6 mesuré par ELISA (Enzyme Linked Ummunosorbent Assay) selon les prescriptions du fournisseur (R&D Systems, Abingdon, GB). La quantité d'IL-6 sécrétée est comparée à celle d'une culture témoin traitée uniquement avec le solvant de la substance C (mélange éthanol/eau (50/50), dilué au 1/100 dans le milieu de culture) et d'une culture stimulée avec un inducteur d'IL-6 de référence, le TNF-α à 50 ng/ml (Fried S. K. J. Clin. Endocrinol. Metab. 1998, 83, 847-860). La figure 1 montre que la substance C stimule la production d'IL-6 des adipocytes 3T3-L1 d'un facteur 5 environ par comparaison avec la culture témoin solvant (43 pg/ml contre 9 pg/ml respectivement), soit 83 % de l'effet obtenu par l'inducteur de référence (52 pg/ml).

Les résultats obtenus montrent que d'une part la substance C reconnaît les adipocytes comme cible d'induction et que d'autre part sous l'effet de la substance C, la production de base de l'IL-6 par les adipocytes est augmentée par un facteur 5 soit 83% de l'effet maximal obtenu avec le témoin positif.

Une telle augmentation permet, compte tenu des propriétés connues de l'IL-6, d'envisager dans un premier temps une réduction de la synthèse de l'enzyme chargée de l'entrée des acides gras, la LPL, puis consécutivement, une réduction de l'entrée des acides gras.

### 2- Effet de la substance C sur la différenciation adipocytaire

Cette deuxième étude avait pour objectif de rechercher les effets de la substance C sur la formation de nouveaux adipocytes.

L'influence de la substance C sur la différenciation des pré-adipocytes 3T3-L1 en adipocytes matures est étudiée d'une part en évaluant la synthèse de l'hormone leptine, un marqueur de la différenciation adipocytaire, et d'autre part en observant au microscope la formation de vésicules lipidiques intra-cytoplasmiques, autres témoins de la transformation fonctionnelle en adipocytes matures. Dans tous les cas, la substance C est introduite dans la culture à 1 % (v/v) au début du processus de différenciation (induit comme précédemment) et maintenue jusqu'à son terme. La synthèse de leptine est évaluée tous les deux jours par dosage ELISA (R8D Systems, Abingdon, GB).

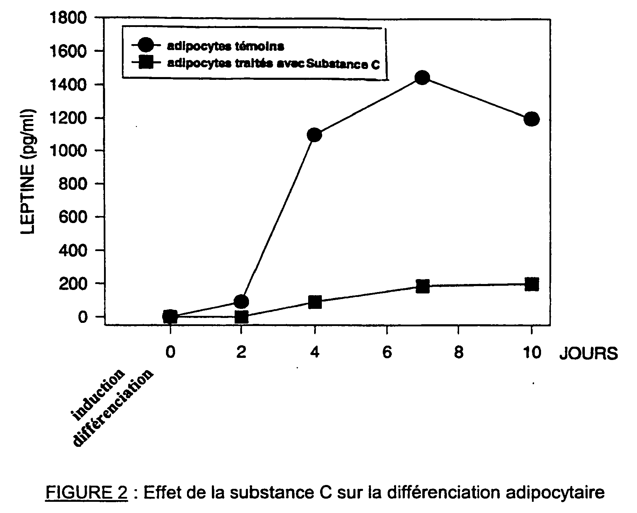

La figure 2 montre qu'en présence de la substance C, la production de leptine reste à un niveau extrêmement faible tout au long des 10 jours d'expérience et très inférieur (≤ 200 pg/ml) à celui observé dans la culture témoin qui, comme attendu, voit son taux de leptine augmenter à partir du 2^{ème} jour suivant l'induction de la différenciation, et croître jusqu'à un plateau maximal de 1200-1400 pg/ml à partir du 6^{ème} jour. L'inhibition de la synthèse de leptine par la substance C constitue la première donnée du blocage du processus de différenciation adipocytaire.

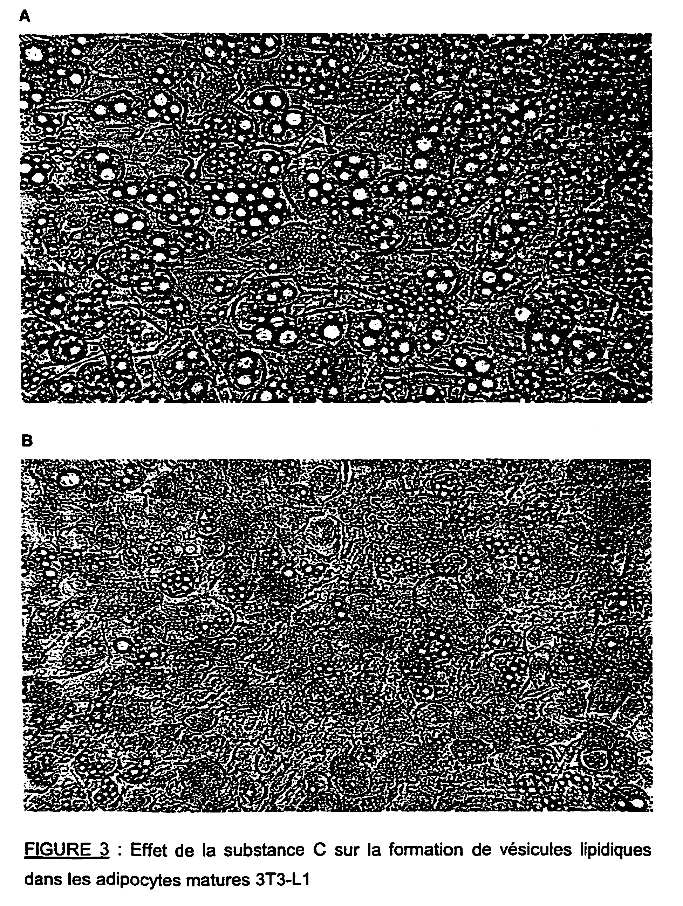

La deuxième est apportée par l'expérience montrée sur la figure 3 qui indique qu'après 10 jours de différenciation, les cellules témoins développent une morphologie d'adipocytes matures renfermant de grandes vésicules lipidiques qui occupent la presque totalité de l'espace cytoplasmique (A), alors que les mêmes cellules traitées par la substance C restent à un stade très immature avec quelques rares vésicules de dimension beaucoup plus faible (B).

En utilisant le modèle 3T3-L1, couramment employé dans la littérature pour l'exploration des fonctions adipocytaires, il a été démontré que la substance C présente la double propriété de stimuler chez des adipocytes matures la production d'IL-6 et d'inhiber chez des cellules précurseurs la transformation en adipocytes matures. La substance C agit donc en amont en prévenant la maturation adipocytaire et en aval en stimulant au niveau des adipocytes matures la production d'une cytokine qui intervient dans la limitation du stockage des acides gras.

Ces résultats viennent à l'appui de la propriété unique de la substance à induire la production de l'IL-6 par les adipocytes dont le fonctionnement autocrine (sur les cellules productrices) et paracrine (sur les cellules à proximité des cellules productrices) s'exerce à la fois sur l'entrée des acides gras dans les adipocytes matures, et sur le recrutement de nouveaux adipocytes, par un arrêt de la différenciation des pré-adipocytes.

Des études cliniques préliminaires ont été réalisées avec des compositions amincissantes selon l'invention qui contiennent les 3 substances actives (ci après nommée composition Am2).

Deux études cliniques ont été réalisées avec la composition Am2.

La première concerne la mesure de l'épaisseur du tissu adipeux sous-cutané, et la seconde utilise la mesure centimétrique.

### 1) Mesure de l'épaisseur du tissu adipeux sous-cutané

Cette étude a pour objectif d'évaluer l'efficacité clinique de la nouvelle composition Am2 contenant les 3 substances actives.

Le protocole utilisé est décrit ci-après.

Nombre de sujets : 61 volontaires féminins sains, répartis en 2 groupes (31 sujets utilisant la composition amincissante Am2 contenant les 3 substances actives, 30 sujets la composition amincissante Am1).

Les produits sont appliqués 2 fois par jour sur l'ensemble des cuisses par un massage circulaire arrêté après la totale pénétration du produit.

L'évaluation de l'efficacité a été déterminée sur une période de 2 mois par la mesure de l'épaisseur du tissu adipeux sous-cutané à T₀, T_{28 jours}, T_{56 jours}. La méthode utilisée est celle de l'échographie.

Le site de mesure est repéré par un marquage cutané et par la hauteur à laquelle est positionnée la sonde.

Pour faciliter le repérage vertical et pour éviter tout mouvement ou compression des tissus lors de la mesure, la sonde échographique est placée sur un dispositif stable, réglable en hauteur et indépendant du manipulateur.

Trois images sont acquises successivement au niveau du repère. Sur chaque cliché, 3 mesures d'épaisseur sont effectuées. L'ensemble des 6 mesures ainsi obtenues permet d'obtenir une précision de ± 1 mm.

Ces mesures sont complétées par une surveillance du poids des sujets inclus dans l'essai et par une analyse des auto-évaluations.

Les significations des résultats sont évaluées au moyen du test t de Student pour des groupes paires. Le test est appliqué aux valeurs brutes ainsi qu'à l'évolution de ces paramètres sur la durée de l'essai (valeur rapportée à T₀).

Quel que soit le groupe considéré, le poids moyen est resté stable pendant toute la durée de l'étude et n'est donc pas susceptible d'être à l'origine des variations de mesure d'épaisseur du tissu adipeux sous-cutané des cuisses.

Les conditions d'étude retenues ne permettent pas de confirmer pour la composition Am1 un effet amincissant significatif : après 1 mois d'application, une diminution de 0,8 % du tissu adipeux sous-cutané est observée et à 2 mois on observe une diminution de 2,6 %.

La composition Am2 permet d'obtenir un effet amincissant très significatif dès le 28^{ème} jour d'application (p<10⁻⁵) : la réduction du tissu adipeux sous-cutané observée avec la composition Am2 est de -2,2 % à 1 mois et de -4,3 % à 2 mois d'application.

### 2) Mesure centimétrique

le protocole ci-après a été mis en oeuvre.

Nombre de sujets : 39 volontaires féminins répartis en 2 groupes : 20 ont reçu la composition Am1 et 19 la composition Am2.

Les produits sont appliqués 2 fois par jour pendant 2 mois.

L'évaluation de l'efficacité amincissante a été évaluée par centimétrie à To, T_{28 ¡ours}, T_{56 jours} au niveau des cuisses (à 3 cm en dessous du pli fessier), droit et gauche. Quel que soit le groupe considéré, le poids moyen est sensiblement constant pendant toute la durée de l'étude et n'est donc pas susceptible d'être à l'origine des variations de mesure d'épaisseur du tissu adipeux sous-cutané des différentes zones étudiées. Le tableau 1 présente les résultats significatifs obtenus par centimétrie.

**Tableau 1 : Résultats obtenus par centimétrie**

| | % de sujets | | Intervalle de diminution | |
|---|---|---|---|---|
| COMPOSITION | 1 mois | 2 mois | 1 mois | 2 mois |
| Composition Am1 | 30 | 45 | 1-2,0 cm | 1-2,0 cm |
| Composition Am2 | 72 | 73 | 1-3,5 cm | 1-2,5 cm |

La composition Am2 a une meilleure activité amincissante *versus* T₀ que la composition Am1 et ce dès un mois d'application.

La comparaison des résultats obtenus à la suite de ces 2 études permet de dégager la nette supériorité de la composition Am2 sur son placebo, et rend compte également de la meilleure performance de la composition Am2 par rapport à la composition Am1.

Ainsi, bien que partageant une partie du mode d'action à travers les substances A + B et leur efficacité sur la lipolyse, l'intensité des bénéfices obtenus avec la nouvelle formule Am2 est renforcée grâce à l'action de la substance C sur l'entrée des acides gras dans les adipocytes.

La combinaison de ces effets, appuyés par l'activité sur le recrutement de nouveaux adipocytes, permet de réduire l'importance du tissu adipeux existant et de prévenir son développement.

Dans la préparation des compositions selon la présente invention, les extraits ainsi constitués sont mélangés aux solvants aqueux ou non et aux diluants conventionnels compatibles avec une utilisation topique ainsi qu'avec les composants actifs de la composition même. Solvants et /ou diluants appropriés seront choisis selon leur capacité à véhiculer le composant actif de l'extrait de l'invention dans la couche adipeuse sous-cutanée.

Ces compositions contiennent généralement des excipients ou des additifs choisis parmi les ingrédients habituellement utilisés dans des compositions destinées à une application locale selon la nécessité des formulations particulières envisagées.

Elles peuvent contenir par exemple, des agents épaississants, des adoucissants, des émollients, des stabilisants, des conservateurs, des agents anti-mousse, des tensioactifs, des antioxydants, des colorants et/ou des pigments, des parfums.

Elles peuvent également contenir d'autres composant actifs ayant soit un effet du même type, par exemple, des produits qui contribuent à la régulation de la lipolyse/lipogénèse ou des produits utiles dans ce type de composition topique tels que des stimulateurs de la synthèse de collagène, des inhibiteurs de la collagénase ou de l'élastase, des vasoprotecteurs.

Les compositions cosmétiques de la présente invention contiennent les substances A, B ou C en pourcentages compris de 0,00001 % à 5 % par rapport au poids total de la composition, en mélange avec les excipients communément utilisés pour la préparation de formulations cosmétiques à appliquer sur la peau.

Lesdits pourcentages peuvent varier dans l'intervalle indiqué ci-dessus en fonction de l'acitivité intrinsèque des composants inclus dans la composition. De préférence les composants A, B et C sont présents en pourcentages de 0,0001 % à 2 %.

Une forme intéressante des compositions selon l'invention est un fluide appliqué topiquement par l'intermédiaire d'un support adhésif, ci-après désigné "patch", ce patch permettant une diffusion contrôlée des composants actifs.

Les compositions de la présente invention jouissent d'une bonne stabilité et peuvent être conservées pendant le temps nécessaire pour l'utilisation à des températures comprises entre 0°C et 60°C sans qu'il y ait sédimentation des constituants ou séparation des phases, ni une diminution de l'activité qui puisse en compromettre l'utilisation.

Ces compositions sont très bien tolérées, elles ne présentent aucune phototoxicité et leur application sur la peau, pour des périodes prolongées n'implique aucun effet secondaire.

Dès les premières applications, le relief cutané est lissé, la peau devient plus tonique et plus ferme. Après un mois d'application, l'effet minceur apparaît, l'aspect « peau d'orange » s'estompe visiblement et la silhouette s'affine.

### EXEMPLE 1

Composition amincissante sous forme de spray patch

| **Matières premières** | **Quantité % en poids** |
|---|---|
| eau déminéralisée | qsp 100 |
| Covacryl AC (polyacrylate de sodium) | 0,8 |
| Covacryl RM (polyacrylate de sodium) | 1,4 |
| PVP K30 (PVP) | 0,2 |
| Covacryl A15 (copolymères d'acrylate) | 7 |
| Covacryl E14 (copolymères d'acrylate) | 3 |
| Covaplast (acétyltributyle citrate/triéthyl citrate/trioctyltriméllitate/éthyllactate) | 2,5 |
| Simulsol 98 (Oleth-20) | 0,5 |
| Dermosoft octiol (ethylhexanediol) | 0.5 |
| Substance A | 0,14 |
| Substance B | 0,007 |
| Substance C | 0,5 |
| Gaz propulseur (butane) | |

### EXEMPLE 2

Composition amincissante sous forme de spray patch

| **Matières premières** | **Quantité % en poids** |
|---|---|
| eau déminéralisée | qsp 100 |
| CMC 7 LF (gomme de cellulose) | 0,5 |
| Natrosol 250 HX (hydroxyéthylcellulose) | 0,3 |
| Aquatrix part B (eau et carboxyméthylchitosan et paraben) | 13 |
| eau déminéralisée | 20 |
| Aquatrix part A (eau déminéralisée et PVP) | 13 |
| Phenonip (phénoxythanol, méthylparaben, propyparaben, butylparaben, éthylparaben) | 1 |
| Simulsol 98 (Oleth-20) | 1 |
| Substance A | 0,08 |
| Substance B | 0,004 |
| Substance C | 0,25 |
| Gaz propulseur (butane) | |

### EXEMPLE 3

Composition amincissante sous forme de gel

| **Matières premières** | **Quantité % en poids** |
|---|---|
| eau déminéralisée | qsp 100 |
| Trilon B (EDTA tétrasodique) | 0,2 |
| Carbopol 2980 (carbomère) | 0,5 |
| Glycérine | 3,0 |
| eau déminéralisée | 20 |
| Lubragel MS (Polyglycérylmétacrylate et propylèneglycol)) | 5,0 |
| Dipropylèneglycol | 3,0 |
| Butylèneglycol | 5,0 |
| Phenonip (phénoxythanol, méthylparaben, propyparaben, butylparaben, éthylparaben) | 0,65 |
| Triéthanolamine | 0,5 |
| Substance A | 0,10 |
| Substance B | 0,01 |
| Substance C | 0,1 |

### EXEMPLE 4

Composition amincissante sous forme d'émulsion

| **Matières premières** | **Quantité % en poids** |
|---|---|
| Eau déminéralisée | qsp 100 g |
| Triéthanolamine | 0,85 |
| Lanette 16 (cétylalcool) | 0,5 |
| Miglyol 812 (caprylic/capric triglycéride) | 2,5 |
| Stéarine TP (acide stéarique) | 1,5 |
| Super Hartolan (lanoline alcool) | 0,2 |
| Silicone DC 200 fluide (diméthicone ou siméthicone SI RAL) | 0,5 |
| Generol 122N (Glycine de soja) | 1,0 |
| Tegin (stearate de glyceryl SE) | 1,0 |
| Myglyol 840 (Propylène glycol dicaprylateledicaprate) | 7,0 |
| Eutanol G (alcool) | 1,5 |
| Montane 60 (Sorbitan stéarate) | 0,43 |
| Montanox 60DF (Polysorbate 60) | 0,57 |
| Carbopol 981 solution à 2% (carbomère) | 13,0 |
| Butylèneglycol | 6,0 |
| Phenonip (phénoxythanol, méthylparaben, propyparaben, butylparaben, éthylparaben) | 1,0 |
| Acétate DL α tocophérol (acétate tocophéryl) | 0,1 |
| Substance A | 0,02 |
| Substance B | 0,001 |
| Substance C | 0,3 |

### EXEMPLE 5

Composition amincissante sous forme d'émulsion gel

| **Matières premières** | **Quantité % en poids** |
|---|---|
| eau | qsq 100 |
| Trilon B (EDTA tétrasodique) | 0,03 |
| Natrosol 250 HX (Hydroxyéthylcellulose) | 0,25 |
| Pémulen TR-1 (copolymère d'acrylates) | 0.,20 |
| Butylèneglycol 1.3 pur | 5 |
| Dipropylèneglycol | 3 |
| Triéthanolamine | 0,2 |
| Phenonip (phénoxythanol, méthylparaben, propyparaben, butylparaben, éthylparaben) | 1 |
| DC 2502 (cétyldiméthicone) | 4 |
| Dermol 105 (isodecyl neopentanoate) | 3 |
| Hyaluronate de sodium | 0,01 |
| Veragel liquide 1:1 (Gel d'Aloe Barbadensis) | 1 |
| Substance A | 0,15 |
| Substance B | 0,1 |
| Substance C | 0,05 |

## Revendications

1. Composition cosmétique amincissante contenant au moins :
- un composant inducteur de la production d'IL-6 par les adipocytes, susceptible d'être obtenu par fermentation de la souche *Rhodotorula sp,* déposée auprès de la C.N.C.M. de l'Institut Pasteur sous le numéro I-1844, ou un de ses mutants producteurs,
- un composant α₂-antagoniste, susceptible d'être obtenu par fermentation de la souche *Bacillus licheniformis,* déposée auprès de la C.N.C.M. de l'institut Pasteur sous la numéro I-1778, ou un de ses mutants producteurs,
- un composant NPY- antagoniste, susceptible d'être obtenu par fermentation de la souche *Streptomyces sp.,* déposée auprès de la C.N.C.M. de l'Institut Pasteur sous le numéro I-1332, ou un de ses mutants producteurs,
en mélange avec un excipient pour préparations cosmétiques.

2. Composition cosmétique amincissante selon la revendication 1, caractérisée en ce ladite composition est à application topique.

## Claims

1. Slimming cosmetic composition containing at least:
- a component which induces IL-6 production by adipocytes, capable of being obtained by fermentation of the *Rhodotorula sp* strain, deposited with the C.N.C.M. [National Collection of Microorganism Cultures] of the Institut Pasteur under number 1-1844, or a producer mutant thereof,
- an α₂-antagonist component, capable of being obtained by fermentation of the *Bacillus licheniformis* strain, deposited with the C.N.C.M. of the Institut Pasteur under number 1-1778, or a producer mutant thereof,
- an NPY-antagonist component, capable of being obtained by fermentation of the *Streptomyces sp.* strain, deposited with the C.N.C.M. of the Institut Pasteur under number 1-1332, or a producer mutant thereof,
as a mixture with an excipient for cosmetic preparations.

2. Slimming cosmetic composition according to Claim 1, **characterized in that** said composition is for topical application.

## Patentansprüche

1. Schlankmachende Kosmetikzusammensetzung, die mindestens Folgendes umfasst:
- einen Bestandteil, der die Produktion von IL-6 durch Adipozyten induziert, welcher durch Fermentation des Stammes *Rhodotorula sp.,* der bei der C.N.C.M. des Institut Pasteur unter der Nummer 1-1844 hinterlegt ist oder einer seiner Produktionsmutanten erhalten werden kann,
- einen Bestandteil eines α₂-Antagonisten, welcher durch Fermentation des Stammes *Bacillus licheniformis,* der bei der C.N.C.M des Institut Pasteur unter der Nummer 1-1778 hinterlegt ist oder einer seiner Produktionsmutanten erhalten werden kann,
- einen Bestandteil eines NPY-Antagonisten, welcher durch Fermentation des Stammes *Streptomyces sp.,* der bei der C.N.C.M. des Institut Pasteur unter der Nummer 1-1332 hinterlegt ist oder einer seiner Produktionsmutanten erhalten werden kann,
im Gemisch mit einem Hilfsstoff für kosmetische Präparate.

2. Schlankmachende Kosmetikzusammensetzung nach Anspruch 1, **dadurch gekennzeichnet, dass** die Zusammensetzung zur topischen Anwendung ist.
